# EUROPEAN PATENT APPLICATION

(11) **EP 1 391 515 A1**
(43) Date of publication of application: **25.02.2004**
(21) Application number: 02722768.5
(22) Date of filing: 25.04.2002
(51) Int. Cl.: C12N 15/54, C12N 9/10, C12N 1/21

(54) **PROCESS FOR PRODUCING COENZYME Q 10**

(30) Priority: 25.04.2001 JP 2001127470
(71) Applicant: KANEKA CORPORATION, Osaka-shi, Osaka 530-8288 (JP)
(72) Inventor: MATSUDA, Hideyuki, Matsue-shi, Shimane 690-0815 (JP); KAWAMUKAI, Makoto, Matsue-shi, Shimane 690-0823 (JP); YAJIMA, Kazuyoshi, Akashi-shi, Hyogo 673-0005 (JP)
(74) Representative: HOFFMANN - EITLE
(86) International application number: PCT/JP2002/004112
(87) International publication number: WO 2002/088365

(57) **Abstract**

The present invention provides a process for producing coenzyme Q₁₀ efficiently in microorganisms by utilizing a coenzyme Q₁₀ side chain synthesis gene from a fungal species belonging to the genus *Bulleromyces*.

The present invention relates to a DNA comprising a base sequence shown under SEQ ID NO:1, a DNA having a base sequence derived from the base sequence shown under SEQ ID NO:1 by deletion, addition, insertion and/or substitution of one or several bases and encoding a protein having decaprenyl diphosphate synthase activity, or a DNA capable of hybridizing with a DNA comprising the base sequence shown under SEQ ID NO:1 under a stringent condition and encoding a protein having decaprenyl diphosphate synthase activity.

SEQUENCE LISTING

<110> KANEKA CORPORATION

<120> PROCESS FOR PRODUCING COENZYME Q10

<130> T656.QX-GT5

<150> JP2001-127470
<151> 2001-04-25

<160> 12

<210> 1
<211> 1584
<212> DNA
<213> Bulleromyces albus

<400> 1
atg ttt cgt tcg gcg cgg gcg gcc act cga gca gct agg cga gct gcc 48
Met Phe Arg Ser Ala Arg Ala Ala Thr Arg Ala Ala Arg Arg Ala Ala
1               5                   10                  15

ggg acc agg tca tca ttg atc aaa tcg aca ccc tca ccg gct tcc gat 96
Gly Thr Arg Ser Ser Leu Ile Lys Ser Thr Pro Ser Pro Ala Ser Asp
            20                  25                  30

gtg gcg aat cca tcc ctc gct cag agg tcg ata cga agt ata tcg acg 144
Val Ala Asn Pro Ser Leu Ala Gln Arg Ser Ile Arg Ser Ile Ser Thr
        35                  40                  45

act cga acg tct tcc tcg cct cag aca gca tgg gcg gaa gcg acg gaa 192
Thr Arg Thr Ser Ser Ser Pro Gln Thr Ala Trp Ala Glu Ala Thr Glu
    50                  55                  60

tcc gca cac tcg atc ttg acc cct ccc acg gcc aat tca ccc tct caa    240
Ser Ala His Ser Ile Leu Thr Pro Pro Thr Ala Asn Ser Pro Ser Gln
65                  70                  75                  80

gat ccc cta gaa gcg ata tac agt gaa atc tca aca tta cgt tcg tcc    288
Asp Pro Leu Glu Ala Ile Tyr Ser Glu Ile Ser Thr Leu Arg Ser Ser
                85                  90                  95

ctg ttc agc atg ctt gga tcg tca cat ccg tct ttg gat aaa gtc gca    336
Leu Phe Ser Met Leu Gly Ser Ser His Pro Ser Leu Asp Lys Val Ala
            100                 105                 110

aag tac tac ttc caa gcg gaa gga aaa cac ctt cgt cca cta ctg gtg    384
Lys Tyr Tyr Phe Gln Ala Glu Gly Lys His Leu Arg Pro Leu Leu Val
        115                 120                 125

cta tta ttg tca caa gcg acg aac ggc cta gcc gga agt gat agc tgg    432
Leu Leu Leu Ser Gln Ala Thr Asn Gly Leu Ala Gly Ser Asp Ser Trp
    130                 135                 140

gag agg gcg aga cat gag gct cag agg agg aat gtg gat gac agt ttg    480
Glu Arg Ala Arg His Glu Ala Gln Arg Arg Asn Val Asp Asp Ser Leu
145                 150                 155                 160

acg agc agg gga ggg gta ttg aat gat tgg aat ccg gaa caa atg ggc    528
Thr Ser Arg Gly Gly Val Leu Asn Asp Trp Asn Pro Glu Gln Met Gly
                165                 170                 175

aaa gag gat cag cag agt aat cca gga gcc gtg ttc gcc aac cca ttc    576
Lys Glu Asp Gln Gln Ser Asn Pro Gly Ala Val Phe Ala Asn Pro Phe
            180                 185                 190

agc ata acg ccc tcg aat cgc tca acc ccg tca tca acg tca tca acg    624
Ser Ile Thr Pro Ser Asn Arg Ser Thr Pro Ser Ser Thr Ser Ser Thr
        195                 200                 205

gct tcg aca gaa gcg cca tcc aat gct tcc tcc tct ctc tcc gac att    672
Ala Ser Thr Glu Ala Pro Ser Asn Ala Ser Ser Ser Leu Ser Asp Ile
    210                 215                 220

ttc aac tcg atg ctt cct tcc tcc tcc tca tac acc gtc cct ctt cct    720
Phe Asn Ser Met Leu Pro Ser Ser Ser Ser Tyr Thr Val Pro Leu Pro
225                 230                 235                 240

ccc tca ctg cag tct tca cct ctg gcc tcg ctg tac agc tcg ccc gga    768
Pro Ser Leu Gln Ser Ser Pro Leu Ala Ser Leu Tyr Ser Ser Pro Gly
                245                 250                 255

acg ccc gag att ctc tcg acg caa aga cgc cta gcg agc atc acc gag    816
Thr Pro Glu Ile Leu Ser Thr Gln Arg Arg Leu Ala Ser Ile Thr Glu
            260                 265                 270

atg atc cac gtt gct tcg tta tta cac gac gac gta att gac aac tcg    864
Met Ile His Val Ala Ser Leu Leu His Asp Asp Val Ile Asp Asn Ser
        275                 280                 285

gca tta cgg cgg aac ctg ccc tct gcc cca tcc gcc ttt ggc tca aaa    912
Ala Leu Arg Arg Asn Leu Pro Ser Ala Pro Ser Ala Phe Gly Ser Lys
    290                 295                 300

ttg tct atc ctc tct ggc gac ttt ttg ctc ggt cgg gca tct gtc gcc    960
Leu Ser Ile Leu Ser Gly Asp Phe Leu Leu Gly Arg Ala Ser Val Ala
305                 310                 315                 320

ctc tcg cgg ctt ggc tcg aat gag gtc gta gag cta ttg gcg acg gtc    1008
Leu Ser Arg Leu Gly Ser Asn Glu Val Val Glu Leu Leu Ala Thr Val
                325                 330                 335

atc gcc aat ctg gtg gaa gga gag gtt ctg caa tta cga gcg acg tcg    1056
Ile Ala Asn Leu Val Glu Gly Glu Val Leu Gln Leu Arg Ala Thr Ser

            340                 345                 350

agc aac gag tcc aca atc gag tgg gat cgg atg ttt gag gag tac atg 1104
Ser Asn Glu Ser Thr Ile Glu Trp Asp Arg Met Phe Glu Glu Tyr Met
        355                 360                 365

agg aag acc tac ttg aag aca gcc agt ctc atg tcg aag tca tgt agg 1152
Arg Lys Thr Tyr Leu Lys Thr Ala Ser Leu Met Ser Lys Ser Cys Arg
    370                 375                 380

gcg gcg gta atc ctc ggt gga tgc gga aga gac att gag agc gaa tgg 1200
Ala Ala Val Ile Leu Gly Gly Cys Gly Arg Asp Ile Glu Ser Glu Trp
385                 390                 395                 400

gtc aag gat gtg gcg tat gga tat gga agg aac ttg ggc att gcg ttc 1248
Val Lys Asp Val Ala Tyr Gly Tyr Gly Arg Asn Leu Gly Ile Ala Phe
                405                 410                 415

cag ctt gtg gac gac gca ctt gac ttt atc ccc gct tcc gac atg ggc 1296
Gln Leu Val Asp Asp Ala Leu Asp Phe Ile Pro Ala Ser Asp Met Gly
            420                 425                 430

aaa ccc tcc gac gga gcc gac cta tca ctc ggt ctc gct acc gca ccc 1344
Lys Pro Ser Asp Gly Ala Asp Leu Ser Leu Gly Leu Ala Thr Ala Pro
        435                 440                 445

gcc ttg ttc gcc tac aag cag aac ccg gcc ttg ggc cct ttg atc ttg 1392
Ala Leu Phe Ala Tyr Lys Gln Asn Pro Ala Leu Gly Pro Leu Ile Leu
    450                 455                 460

cgg aag ttc gag ggt gaa ggg gat gtt caa gct gcc aag aag atg atc 1440
Arg Lys Phe Glu Gly Glu Gly Asp Val Gln Ala Ala Lys Lys Met Ile
465                 470                 475                 480

atg gag tct gat ggt gtg aag gag acg ata aac ctt gca aag aaa ttt 1488

Met Glu Ser Asp Gly Val Lys Glu Thr Ile Asn Leu Ala Lys Lys Phe
                485                 490                 495

gca aac tcg gca aag gac ttg tta gag cta tta cca gag agc gag gct 1536
Ala Asn Ser Ala Lys Asp Leu Leu Glu Leu Leu Pro Glu Ser Glu Ala
            500                 505                 510

aga ggg gcg ttg gtc ggc ctc acg aag aag gtt gtg gaa aga gtg 1581
Arg Gly Ala Leu Val Gly Leu Thr Lys Lys Val Val Glu Arg Val
        515                 520                 525

aag 1584
Lys

<210> 2
<211> 528
<212> PRT
<213> Bulleromyces albus

<400> 2
Met Phe Arg Ser Ala Arg Ala Ala Thr Arg Ala Ala Arg Arg Ala Ala
1               5                   10                  15

Gly Thr Arg Ser Ser Leu Ile Lys Ser Thr Pro Ser Pro Ala Ser Asp
            20                  25                  30

Val Ala Asn Pro Ser Leu Ala Gln Arg Ser Ile Arg Ser Ile Ser Thr
        35                  40                  45

Thr Arg Thr Ser Ser Ser Pro Gln Thr Ala Trp Ala Glu Ala Thr Glu
    50                  55                  60

Ser Ala His Ser Ile Leu Thr Pro Pro Thr Ala Asn Ser Pro Ser Gln
65                  70                  75                  80

Asp Pro Leu Glu Ala Ile Tyr Ser Glu Ile Ser Thr Leu Arg Ser Ser
                85                  90                  95

Leu Phe Ser Met Leu Gly Ser Ser His Pro Ser Leu Asp Lys Val Ala
            100                 105                 110

Lys Tyr Tyr Phe Gln Ala Glu Gly Lys His Leu Arg Pro Leu Leu Val
        115                 120                 125

Leu Leu Leu Ser Gln Ala Thr Asn Gly Leu Ala Gly Ser Asp Ser Trp
    130                 135                 140

Glu Arg Ala Arg His Glu Ala Gln Arg Arg Asn Val Asp Asp Ser Leu
145                 150                 155                 160

Thr Ser Arg Gly Gly Val Leu Asn Asp Trp Asn Pro Glu Gln Met Gly
                165                 170                 175

Lys Glu Asp Gln Gln Ser Asn Pro Gly Ala Val Phe Ala Asn Pro Phe
            180                 185                 190

Ser Ile Thr Pro Ser Asn Arg Ser Thr Pro Ser Ser Thr Ser Ser Thr
        195                 200                 205

Ala Ser Thr Glu Ala Pro Ser Asn Ala Ser Ser Ser Leu Ser Asp Ile
    210                 215                 220

Phe Asn Ser Met Leu Pro Ser Ser Ser Ser Tyr Thr Val Pro Leu Pro
225                 230                 235                 240

Pro Ser Leu Gln Ser Ser Pro Leu Ala Ser Leu Tyr Ser Ser Pro Gly
                245                 250                 255

Thr Pro Glu Ile Leu Ser Thr Gln Arg Arg Leu Ala Ser Ile Thr Glu

            260                 265                 270

Met Ile His Val Ala Ser Leu Leu His Asp Asp Val Ile Asp Asn Ser
        275                 280                 285

Ala Leu Arg Arg Asn Leu Pro Ser Ala Pro Ser Ala Phe Gly Ser Lys
    290                 295                 300

Leu Ser Ile Leu Ser Gly Asp Phe Leu Leu Gly Arg Ala Ser Val Ala
305                 310                 315                 320

Leu Ser Arg Leu Gly Ser Asn Glu Val Val Glu Leu Leu Ala Thr Val
                325                 330                 335

Ile Ala Asn Leu Val Glu Gly Glu Val Leu Gln Leu Arg Ala Thr Ser
            340                 345                 350

Ser Asn Glu Ser Thr Ile Glu Trp Asp Arg Met Phe Glu Glu Tyr Met
        355                 360                 365

Arg Lys Thr Tyr Leu Lys Thr Ala Ser Leu Met Ser Lys Ser Cys Arg
    370                 375                 380

Ala Ala Val Ile Leu Gly Gly Cys Gly Arg Asp Ile Glu Ser Glu Trp
385                 390                 395                 400

Val Lys Asp Val Ala Tyr Gly Tyr Gly Arg Asn Leu Gly Ile Ala Phe
                405                 410                 415

Gln Leu Val Asp Asp Ala Leu Asp Phe Ile Pro Ala Ser Asp Met Gly
            420                 425                 430

Lys Pro Ser Asp Gly Ala Asp Leu Ser Leu Gly Leu Ala Thr Ala Pro
        435                 440                 445

Ala Leu Phe Ala Tyr Lys Gln Asn Pro Ala Leu Gly Pro Leu Ile Leu
    450                 455                 460

Arg Lys Phe Glu Gly Glu Gly Asp Val Gln Ala Ala Lys Lys Met Ile
465                 470                 475                 480

Met Glu Ser Asp Gly Val Lys Glu Thr Ile Asn Leu Ala Lys Lys Phe
                485                 490                 495

Ala Asn Ser Ala Lys Asp Leu Leu Glu Leu Leu Pro Glu Ser Glu Ala
            500                 505                 510

Arg Gly Ala Leu Val Gly Leu Thr Lys Lys Val Val Glu Arg Val Lys
        515                 520                 525

<210> 3
<211> 24
<212> DNA
<213> Artificial Sequence

<220>
<223> Description of Artificial Sequence: primer DPS-1

<400> 3
aaggatcctn ytncaygayg aygt                                        24

<210> 4
<211> 17
<212> DNA
<213> Artificial Sequence

<220>
<223> Description of Artificial Sequence: primer DPS-1 1AS

<400> 4
arytgnadra aytcncc                                                    17

<210> 5
<211> 21
<212> DNA
<213> Artificial Sequence

<220>
<223> Description of Artificial Sequence: primer B1S

<400> 5
tcggcattac ggcggaacct g                                               21

<210> 6
<211> 15
<212> DNA
<213> Artificial Sequence

<220>
<223> Description of Artificial Sequence: primer B7ASP

<400> 6
cacaccatca gactc                                                      15

<210> 7
<211> 20
<212> DNA
<213> Artificial Sequence

<220>

<223> Description of Artificial Sequence: primer B5S

<400> 7
tgggtcaagg atgtggcgta                                                    20

<210> 8
<211> 21
<212> DNA
<213> Artificial Sequence

<220>
<223> Description of Artificial Sequence: primer B4AS

<400> 8
cagattggcg atgaccgtcg c                                                  21

<210> 9
<211> 21
<212> DNA
<213> Artificial Sequence

<220>
<223> Description of Artificial Sequence: primer B6S

<400> 9
gttcgcctac aagcagaacc c                                                  21

<210> 10
<211> 20
<212> DNA
<213> Artificial Sequence

<220>
<223> Description of Artificial Sequence: primer B3AS

<400> 10
ttgagccaaa ggcggatggg                                                20

<210> 11
<211> 28
<212> DNA
<213> Artificial Sequence

<220>
<223> Description of Artificial Sequence: primer BN1

<400> 11
aaggatccat atgtttcgtt cggcgcgg                                       28

<210> 12
<211> 26
<212> DNA
<213> Artificial Sequence

<220>
<223> Description of Artificial Sequence: primer BCH

<400> 12
ccaagcttct acttcactct ttccac                                         26

## Description

### TECHNICAL FIELD

The present invention relates to a process for producing coenzyme Q₁₀, which is in use as a drug or the like. More particularly, it relates to a process for causing formation of coenzyme Q₁₀ by isolating a gene coding for a coenzyme Q₁₀ side chain synthase, which serves as a key enzyme in the biosynthesis of coenzyme Q₁₀, namely a decaprenyl diphosphate synthase, from a fungal species belonging to the genus *Bulleromyces* and introducing that gene into a microorganism.

### BACKGROUND ART

An industrial process for producing coenzyme Q₁₀, which is conventional in the art, comprises, for example, isolating coenzymes of plant origin, for example of tobacco origin, and adjusting the side chain length thereof by a synthetic method.

It is known that coenzyme Q₁₀ is produced in a wide variety of organisms, from microorganisms, such as bacteria and yeasts, to higher animals and plants. Thus, the process comprising cultivating a microorganism and extracting this substance from cells thereof can be regarded as one of the most efficient processes for producing coenzyme Q₁₀ and has actually been employed in commercial production thereof. However, the productivity of such processes can hardly be said to be good, since the productivity is low and the procedure is complicated, for instance.

Attempts have also been made to increase the production of coenzyme Q₁₀ by isolating genes involved in the biosynthesis of coenzyme Q₁₀ and amplifying the genes utilizing the recombinant DNA technology. Coenzyme Q₁₀ is formed *in vivo* in a multistage process comprising complicated reactions in which a number of enzymes are involved. The route of biosynthesis thereof in prokaryotes partially differs from that in eukaryotes. Basically, however, each route comprises three fundamental steps, namely the step of synthesis of decaprenyl diphosphate, which is the source of the prenyl side chain of coenzyme Q₁₀, the step of synthesis of para-hydroxybenzoic acid, which is the source of the quinone ring, and the step of completion of coenzyme Q₁₀ through coupling of these two compounds and successive substituent conversions. Among these reactions, the reaction which determines the side chain length of coenzyme Q₁₀, namely the decaprenyl diphosphate synthase-involving reaction, which is said to be a rate-determining one in the whole biosynthetic reaction route, is considered to be the most important one.

Therefore, for efficient production of coenzyme Q₁₀, it is considered effective to isolate a decaprenyl diphosphate synthase gene, which is the key gene in the biosynthesis of coenzyme Q₁₀, and utilize the same for the purpose of increasing production. As for the gene source, fungi, in which coenzyme Q₁₀ is produced in relatively large amounts, are leading candidates.

So far, decaprenyl diphosphate synthase genes have been isolated from several microorganisms, such as *Schizosaccharomyces pombe* (JP-A-09-173076) and *Gluconobacter suboxydans* (JP-A-10-57072). However, the productivity of coenzyme Q₁₀ in these microorganisms cannot be said to be satisfactory, and the cultivation of these microorganisms and the separation/purification of coenzyme Q₁₀ therefrom have not been efficient. It has thus been desired that a microorganism-derived gene for that enzyme, which enables high level production of coenzyme Q₁₀, be isolated. As regards fungus-derived decaprenyl diphosphate synthase genes, the isolation of such a gene from a microorganism belonging to the genus *Saitoella* has been reported (WO 00/5659). However, there is no report available about the isolation of such an enzyme gene from a microorganism belonging to the genus *Bulleromyces*.

### SUMMARY OF THE INVENTION

It is an object of the present invention to produce coenzyme Q₁₀ efficiently in microorganisms by isolating a coenzyme Q₁₀ side chain synthesis gene from a fungal species belonging to the genus *Bulleromyces* and utilizing the same to thereby solve the above-mentioned productivity problem.

For attaining the above object, in the present inventioin, firstly, investigations were made in an attempt to isolate a key gene involved in the biosynthesis of coenzyme Q₁₀, a decaprenyl diphosphate synthase gene from fungi belonging to the genus *Bulleromyces* and, as a result, succeeded in isolating such gene. This success has now led to completion of the present invention.

Thus, the present invention relates to
a DNA of the following (a), (b) or (c):
(a) a DNA whose base sequence is as described under SEQ ID NO:1;
(b) a DNA having a base sequence derived from the base sequence shown under SEQ ID NO:1 by deletion, addition, insertion and/or substitution of one or several bases and
   encoding a protein having decaprenyl diphosphate synthase activity;
(c) a DNA capable of hybridizing with a DNA comprising the base sequence shown under SEQ ID NO:1 under a stringent condition and
   encoding a protein having decaprenyl diphosphate synthase activity.

The invention further relates to
a protein of the following (d) or (e):
(d) a protein whose amino acid sequence is as described under SEQ ID NO:2;
(e) a protein having an amino acid sequence derived from the amino acid sequence shown under SEQ ID NO:2 by deletion, addition, insertion and/or substitution of one or several amino acid residues and having decaprenyl diphosphate synthase activity.

The invention further relates to
DNAs respectively encoding the proteins (d) or (e).

Furthermore, the invention relates to
an expression vector resulting from insertion of the DNA mentioned above into a vector for expression,
the above expression vector,
in which the vector for expression is pUCNT, and
the above expression vector,
which is pNTB1.

The invention further relates to
a transformant resulting from transformation of a host microorganism with the DNA mentioned above,
a transformant resulting from transformation of a host microorganism with the above expression vector,
the above transformant,
wherein the host microorganism is *Escherichia coli*,
the above transformant,
wherein the *Escherichia coli* is *Escherichia coli* DH5α, and
the above transformant,
which is *Escherichia coli* DH5α(pNTB1)(FRPM BP-7512).

The invention still further relates to
a process for producing coenzyme Q₁₀,
which comprises cultivating the transformant in a medium and recovering coenzyme Q₁₀ thus formed and accumulated in the medium.

### DETAILED DISCLOSURE OF THE INVENTION

The present inventors made investigations to isolate a gene encoding the enzyme in question from fungi belonging to the genus *Bulleromyces*, in which coenzyme Q₁₀ is produced in relatively large amounts, and, as a result, succeeded in obtaining a fragment of the gene by PCR method.

First, the sequence of a known gene encoding a decaprenyl diphosphate synthase was compared with that of a known gene encoding a polyprenyl diphosphate synthase which is an analogue of the enzyme in question and a long prenyl chain synthase of different chain length coenzyme Q, and various PCR primers were synthesized for regions showing high homology therebetween. PCR conditions were studied for various combinations of these primers and, as a result, it was revealed, by gene base sequence analysis, that when 40 PCR cycles, each comprising 94°C, 1 minute → 43°C, 2 minutes → 72°C, 2 minutes, are carried out after 3 minutes of heat treatment at 94°C, using the primers DPS-1 (SEQ ID NO:3 in the sequence listing) and DPS-1 1AS (SEQ ID NO:4 in the sequence listing), a fragment, about 220 bp in size, of the enzyme gene in question is amplified from the chromosomal gene of *Bulleromyces albus* IFO 1192, which is a fungal species belonging to the genus *Bulleromyces*.

Then, for obtaining the enzyme gene in its full length, mRNA was prepared from cells of *Bulleromyces albus* IFO 1192, a 5' terminal side fragment of that enzyme gene was obtained by preparing a primer based on the inner sequence previously obtained and utilizing the 5' RACE method, and a 3' terminal side fragment was obtained by conducting RT-PCR using oligo dT primer relative to a poly A sequence, which is specific for the mRNA. Further, primers were prepared based on the inner sequences of the respective fragments obtained, and PCR was conducted using them, whereby a DNA fragment supposedly comprising the full-length enzyme gene in question was obtained.

The base sequence of the DNA fragment obtained was determined, whereupon it was revealed that it has the sequence shown under SEQ ID NO:1 in the sequence listing. In the amino acid sequence predicted from this base sequence, there was found a sequence characteristic of decaprenyl diphosphate synthase gene.

The DNA of the present invention may be a DNA whose base sequence is as described under SEQ ID NO:1, or a DNA having a base sequence derived from the base sequence shown under SEQ ID NO:1 by deletion, addition, insertion and/or substitution of one or several bases and encoding a protein having decaprenyl diphosphate synthase activity, or a DNA capable of hybridizing with a DNA comprising the base sequence shown under SEQ ID NO:1 under a stringent condition and encoding a protein having decaprenyl diphosphate synthase activity.

A number of amino acids each may be encoded by one or more codons (genetic code degeneracy) , so that a number of DNAs other than the DNA having the base sequence shown under SEQ ID NO:1 can encode the protein having the amino acid sequence shown under SEQ ID NO:2. Therefore, the DNA of the invention includes such DNAs encoding the protein having the amino acid sequence shown under SEQ ID NO:2 as well.

The expression "the base sequence derived by deletion, addition, insertion and/or substitution of one or several bases" as used herein means a base sequence resulting from deletion, addition, insertion and/or substitution of such a number of bases as can be deleted, added, inserted and/or substituted according to the methods well known to those skilled in the art, for example those as described in Supplemental Issue, Tanpakushitsu, Kakusan, Koso (Protein, Nucleic Acid and Enzyme) , PCR Method for Gene Amplification, TAKKAJ, 35 (17), 2951-3178 (1990) or Henry A. Erlich (ed.), translated into Japanese under the supervision of Ikunoshin Kato: PCR Technology (1990).

The expression "DNA capable of hybridizing with a DNA comprising the base sequence shown under SEQ ID NO:1 under a stringent condition" means a DNA obtainable by utilizing the technique of colony hybridization, plaque hybridization or southern hybridization, among others, using a DNA comprising the base sequence shown under SEQ ID NO:1 as a probe. Those skilled in the art would be able to readily obtain the desired DNA by carrying out such hybridization according to the method described in Molecular Cloning, 2nd Edt. (Cold Spring Harbor Laboratory Press, 1989). For example, the desired DNA can be obtained by carrying out such hybridization at a temperature of 50°C or above and at a salt concentration of urea-free SSC of not higher than 0.5 M.

The expression "protein having decaprenyl diphosphate synthase activity" means a protein capable of synthesizing decaprenyl diphosphate in a yield of not less than 10%, preferably not less than 40%, more preferably not less than 60%, still more preferably not less than 80%, as compared with the case where a protein having the amino acid sequence shown under SEQ ID NO:2 is used. Such capacity can be measured by reacting FPP (farnesyl diphosphate) with ¹⁴C-IPP (radiolabeled isopentenyl diphosphate) in the presence of the enzyme in question, hydrolyzing the resulting ¹⁴C-DPP (decaprenyl diphosphate) with phosphatase and, after separation by TLC, determining the incorporation in each spot for each chain length (Okada et al., Eur. J. Biochem., 255, 52-59).

The protein of the invention may be a protein whose amino acid sequence is as described under SEQ ID NO:2, or a protein having an amino acid sequence derived from the amino acid sequence shown under SEQ ID NO:2 by deletion, addition, insertion and/or substitution of one or several amino acid residues and having decaprenyl diphosphate synthase activity.

Such "an amino acid sequence derived by deletion, addition, insertion and/or substitution of one or several amino acid residues" can be obtained by deleting, adding, inserting and/or substituting such a number of amino acid residues as can be deleted, added, inserted and/or substituted by site-directed mutagenesis or any other methods well known in the art. Such methods are specifically described, for example, in Nucleic Acids Res., 10, 6487 (1982) and Methods in Enzymology, 100, 448 (1983).

For causing expression of the decaprenyl diphosphate synthase gene, it is necessary to connect that gene to a site downstream of an appropriate promoter. It is possible to construct an expression vector, for example, by excising a DNA fragment containing the gene using a restriction enzyme or amplifying an enzyme-encoding gene portion alone by PCR and then inserting the fragment or amplification product into a promoter-containing vector.

In the practice of the invention, the vector in which a DNA encoding a protein having decaprenyl diphosphate synthase activity is to be inserted to give an expression vector is not particularly restricted but may be one derived from an *Escherichia coli*-derived plasmid with an appropriate promoter inserted therein. The *Escherichia coli*-derived plasmid includes, among others, pBR322, pBR325, pUC19, pUC18, and pUC119. The promoter includes, among others, the T7 promoter, trp promoter, tac promoter, lac promoter, and λPL promoter.

In the practice of the invention, pGEX-2T, pGEX-3T, pGEX-3X (the three being products of Pharmacia), pBluescriptII, pUC19, pUC18 (product of Toyobo Co., Ltd.), pMALC2, pET-3T, pUCNT (described in WO 94/03613) and the like may also be used as vectors for expression. Among these, pUCNT is suitably used. In a specific example, a decaprenyl diphosphate synthase gene expression vector can be constructed by inserting a gene having the base sequence shown under SEQ ID NO:1 into the vector for expression pUCNT.

And, by introducing the above DNA or expression vector into an appropriate host microorganism, it becomes possible to utilize the microorganism for the production of coenzyme Q₁₀. In,other words, the DNA or expression vector can be utilized in the production of coenzyme Q₁₀ in the form of a transformant produced by transforming a host microorganism therewith.

The host microorganism is not particularly restricted but *Escherichia coli* and the like are suitably used. The *Escherichia coli* strain is not particularly restricted but includes XL1-Blue, BL-21, JM109, NM522, DH5α, HB101, and DH5, among others. Among them, *Escherichia coli* DH5α is suitably used. For example, when a decaprenyl diphosphate synthase gene expression vector pNTB1 is introduced into *Escherichia coli,* this can be transformed so that coenzyme Q₁₀, which *Escherichia coli* originally does not produce, can be produced. This *Escherichia coli* strain *E. coli* DH5α(pNTB1) has been internationally deposited as of March 16, 2001, under the Budapest Treaty, with the National Institute of Advanced Industrial Science and Technology International Patent Organism Depositary, Central 6, 1-1-1 Higashi, Tsukuba, Ibaraki, Japan, under the accession number FERM BP-7512.

Coenzyme Q₁₀ can be produced by cultivating the transformant obtained in the present invention in a medium in the conventional manner and recovering coenzyme Q₁₀ from the cultivation product.

In cases where the host microorganism is *Escherichia coli,* LB medium, or M9 medium containing glucose and casamino acids, for instance, can be used as the medium. For better promoter functioning, such an agent as isopropylthiogalactoside or indolyl-3-acrylic acid, for instance, may be added to the medium. The cultivation is carried out, for example, at 20 to 40°C, preferably 30 to 37°C, more preferably 37°C, for 17 to 24 hours, if necessary with aeration and/or agitation.

In the practice of the invention, the product coenzyme Q₁₀ obtained may be purified or used in the form of a crude product according to the selection duly made depending on the intended use thereof. Coenzyme Q₁₀ can be isolated from the cultivation product by an appropriate combination of *per se* known methods of separation and/or purification. The *per se* known methods of separation and/or purification include salting out, solvent precipitation and other methods utilizing the difference in solubility; dialysis, ultrafiltration, gel filtration, (SDS-)polyacrylamide gel electrophoresis and other methods mainly utilizing the difference in molecular weight; ion exchange chromatography and other methods utilizing the difference in charge; affinity chromatography and other methods utilizing specific affinity; reversed phase high-performance liquid chromatography and other methods utilizing the difference in hydrophobicity; isoelectric focusing and other methods utilizing the difference in isoelectric point, among others.

The field of utilization of coenzyme Q₁₀ obtained in the present invention is not particularly restricted but it may be suitably used as a drug, among others.

### BRIEF DESCRIPTION OF THE DRAWINGS

Fig. 1 is a restriction enzyme map of the expression vector pNTB1.
Fig. 2 shows HPLC analysis charts showing production of coenzyme Q₁₀ by recombinant *E. coli* DH5α(pNTB1).

### BEST MODES FOR CARRYING OUT THE INVENTION

The following examples illustrate the present invention in more detail. These examples are, however, by no means limitative of the scope of the invention.

### (Example 1)

The chromosomal DNA of *Bulleromyces albus* IFO 1192 was prepared by the method of C. S. Hoffman et al. (Gene, 57 (1987), 267-272). Based on the homology with the known long-chain prenyl diphosphate synthase gene, primers for use in PCR, namely DPS-1 (SEQ ID NO:3 in the sequence listing) and DPS-1 1AS(SEQ ID NO: 4 in the sequence listing), were designed. Using these, a PCR cycle of 94°C, 1 minute → 43°C, 2 minutes → 72°C, 2 minutes, was repeated 40 times after 3 minutes of heat treatment at 94°C, followed by 1.2% agarose gel electrophoresis.

The thus-obtained fragment, about 220 bp in size, was purified by excising the corresponding gel portion from the gel and then treating it with a DNA extraction kit (Sephaglas (trademark) BrandPrep Kit, product of Amersham Pharmacia Biotech), and the purified fragment was cloned into a vector for expression in *Escherichia coli* using a PCR product direct cloning kit (pT7 BlueT-Vector Kit, product of NOVAGEN) to give pT7-B1DPS. The DNA base sequence was determined by carrying out the reaction on a DNA sequencer (model 377, product of Perkin Elmer) using a DNA sequencing kit (product of Perkin Elmer, ABI PRISM (trademark) BigDye (trademark) Terminator Cycle Sequence Ready Reaction Kit with AmptiTaq (registered trademark) DNA polymerase, FS) and according to the manual attached thereto. As a result, a DNA fragment having a base sequence covering the 835th to 1,044th bases of the base sequence shown under SEQ ID NO:1 in the sequence listing were obtained from *Bulleromyces albus* IFO 1192. These DNA fragments were estimated to be part of the decaprenyl diphosphate synthase gene since the amino acid sequence "GDFLLXRA"(X is A or G), which is a characteristic region of the long-chain prenyl chain-containing prenyl diphosphate synthase, could be found in the sequence translated from the base sequence determined in the above manner.

### (Example 2)

*Bulleromyces albus* IFO 1192 was cultured in 50 mL of 703 medium (5g/L pepton, 3g/L yeast extract, 3g/L malt extract, and 1g/L glucose, pH 6.0) for 48 hours at 25°C. Then, the cells were harvested by centrifugation at 3,000 revolutions for 20 minutes and frozen immediately in liquid nitrogen.

The frozen cells were placed in a mortar chilled previously at -70°C and ground to a powder with a pestle while being kept frozen by occasionally adding liquid nitrogen. Total RNA was prepared from the well-powdered cells using an RNeasy Maxi RNA Purification Kit (product of Qiagen K.K.). Total RNA thus extracted was further purified using an RNeasy Mini RNA Purification Kit (product of Qiagen K.K.). From the purified total RNA, mRNAwas prepared using an mRNA purification kit (Ologotex-dT30 <Super> (trademark) mRNA Purification kit, product of Takara Shuzo Co., Ltd.).

### (Example 3)

The DNA fragment containing the region from the DNA obtained in Example 1 to the 3'-terminal region of the decaprenyl diphosphate synthase gene of *Bulleromyces albus* IFO 1192 was obtained. RT-PCR was carried out using an RT-PCR kit (High fidelity RNA PCR Kit, product of Takara Shuzo Co., Ltd.) and primer B1S(SEQ ID NO:5 in the sequence listing) which was generated based on the inner sequence of the DNA fragment obtained in Example 1.

The thus-obtained fragment, about 850 bp in size, was purified by excising the corresponding gel portion from the gel and then treating with a DNA extraction kit (Sephaglas (trademark) BrandPrep Kit, product of Amersham Pharmacia Biotech), and the purified fragment was cloned into a vector for expression in *Escherichia coli* using a PCR product direct cloning kit (pT7 BlueT-Vector Kit, product of NOVAGEN) to give pT7-B2DPS. The DNA base sequence was determined by carrying out the reaction on a DNA sequencer (model 377, product of Perkin Elmer) using a DNA sequencing kit (product of Perkin Elmer, ABI PRISM (trademark) BigDye (trademark) Terminator Cycle Sequence Ready Reaction Kit with AmptiTaq (registered trademark) DNA polymerase, FS) and according to the manual attached thereto.

### (Example 4)

The DNA fragment containing the region from the DNA obtained in Example 1 to the 5'-terminal region of the decaprenyl diphosphate synthase gene of *Bulleromyces albus* IFO 1192 was obtained. Reverse transcription was carried out using a 5'-Full RACE Core Set (product of Takara Shuzo Co., Ltd.), the mRNA prepared in Example 2 as a template, and primer B7ASP (SEQ ID NO:6 in the sequence listing, "5'-terminal region-phosphorylated") , which was generated based on the inner sequence of the DNA fragment obtained in Example 3, to synthesize a cDNA encoding the 5'-terminal region of the gene containing a part of the fragment obtained in Example 3. Then, the cDNA was circularized using the above kit.

PCR was carried out using this circularized cDNA as a template, and primers B5S (SEQ ID NO:7 in the sequence listing) and B4AS(SEQ ID NO:8 in the sequence listing), which were generated based on the known part of the sequence obtained in Example 3. Furthermore, PCR was carried out to the PCR product using primers B6S(SEQ ID NO:9 in the sequence listing) and B3AS (SEQ ID NO: 10 in the sequence listing) to obtain a fragment, about 950 bp in size. The thus-obtained fragment, about 950 bp in size, was purified by excising the corresponding gel portion from the gel and then treating with a DNA extraction kit (Sephaglas (trademark) BrandPrep Kit, product of Amersham Pharmacia Biotech), and the purified fragment was cloned into a vector for expression in *Escherichia coli* using a PCR product direct cloning kit (pT7 BlueT-Vector Kit, product of NOVAGEN) to give pT7-B3DPS. The DNA base sequence was determined by carrying out the reaction on a DNA sequencer (model 310, product of Perkin Elmer) using a DNA sequencing kit (product of Perkin Elmer, ABI PRISM (trademark) BigDye (trademark) Terminator Cycle Sequence Ready Reaction Kit with AmptiTaq (registered trademark) DNA polymerase, FS) and according to the manual attached thereto.

### (Example 5)

To obtain the full-length sequence of the decaprenyl diphosphate synthase gene of *Bulleromyces albus* IFO 1192, PCR was carried out using pT7-B2DPS as a template, primer BN1(SEQ ID NO:11 in the sequence listing), which was generated based on the 5'-terminal sequence of the gene and primer B3AS (SEQ ID NO:10 in the sequence listing) described above to obtain the 5'-terminal region, about 910 bp in size. And, PCR was carried out using pT7-B3DPS as a template, primer B1S(SEQ ID NO:5 in the sequence listing) described above and primer BCH(SEQ ID NO:12 in the sequence listing), which was generated based on the 3'-terminal sequence of the gene, to obtain the 3' -terminal region, about 750 bp in size. The two fragments were mixed and denatured, and then slowly cooled to allow annealing, followed by double-strand synthesis using DNA polymerase. Subsequently, PCR was carried out using the double-stranded DNA as a template, and primers BN1 and BCH to obtain DNA containing the full-length sequence of the gene. The base sequence was determined using a DNA sequencer (model 310, product of Perkin Elmer) and a DNA sequencing kit (product of Perkin Elmer, ABI PRISM (trademark) BigDye (trademark) Terminator Cycle Sequence Ready Reaction Kit with AmptiTaq (registered trademark) DNA polymerase, FS) and according to the manual attached thereto. Thus, the total sequence of the decaprenyl diphosphate synthase gene of *Bulleromyces albus* IFO 1192 was revealed. Base sequence was determined on the DNA, about 1.6 kbp in size, and the result is shown under SEQ ID NO:1 in the sequence listing. The amino acid sequence deduced from this base sequence is shown under SEQ ID NO:2.

### (Example 6)

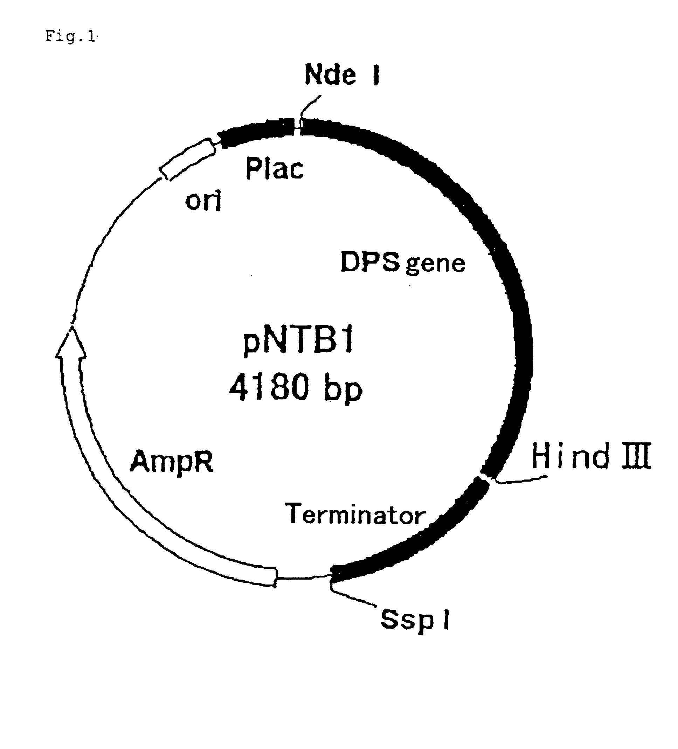

The DNA obtained in Example 4 was cleaved with the restriction enzymes *Nde*I and *Hind*III, and the cleavage product was inserted into a vector for expression, pUCNT (described in WO 94/03613), to give a decaprenyl diphosphate synthase gene expression vector, pNTB1. The restriction enzyme map of the thus-obtained expression vector pNTB1 is shown in Fig. 1. The symbol DPS stands for the coding region of the decaprenyl diphosphate synthase gene.

### (Example 7)

The decaprenyl diphosphate synthase gene expression vector pNTB1 was introduced into *Escherichia coli* DH5α to produce recombinant *Escherichia coli, E. coli* DH5α(pNTB1). The recombinant *Escherichia coli* was shake-cultured overnight in 10 mL of LB medium at 37°C, and cells were harvested by centrifugation (3,000 revolutions, 20 minutes).

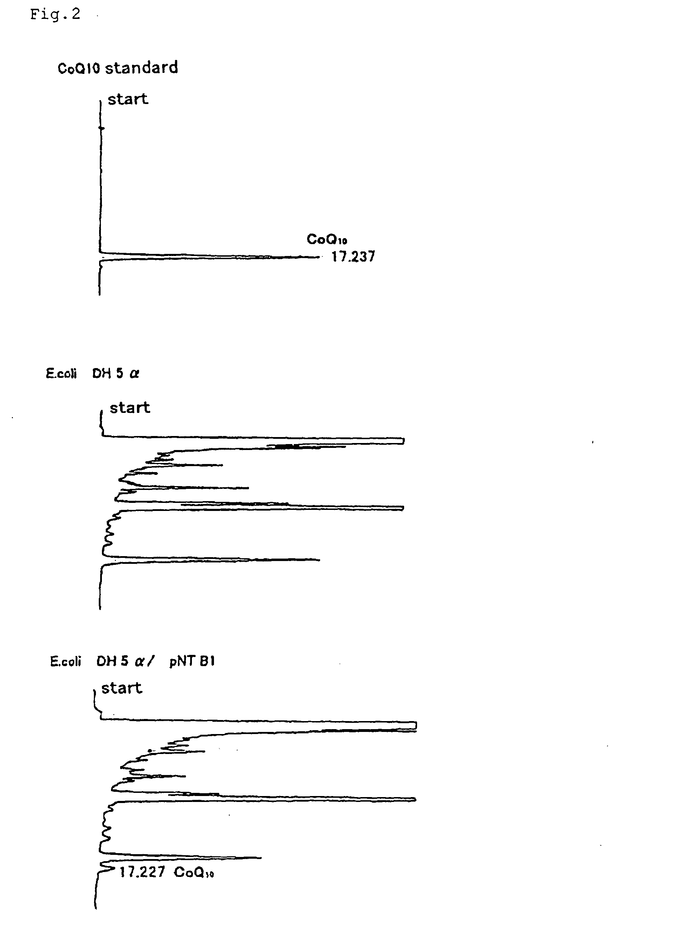

The cells were suspended in 1 mL of a 3% aqueous solution of sulfuric acid and, after 30 minutes of heat treatment at 120°C, 2 mL of a 14% aqueous solution of sodium hydroxide was added, followed by further 15 minutes of heat treatment at 120°C. To the thus-treated suspension was added 3 mL of hexane-isopropanol (10:2) for effecting extraction. After centrifugation, 1.5 mL of the organic solvent layer was separated, and the solvent was evaporated to dryness under reduced pressure conditions. The residue was dissolved in 200 µL of ethanol, and 20 µL of the solution was analyzed by high-performance liquid chromatography (using LC-10A, product of Shimadzu Corp.). For separation, a reversed phase column (YMC-pack ODS-A, 250 x 4. 6 mm, S-5 µm, 120A) was used, and the coenzyme Q₁₀ formed was detected based on the absorbance at the wavelength 275 nm. An HPLC chart (analysis performed using the mobile phase: ethanol/methanol = 2:1) showing production of coenzyme Q₁₀ in the recombinant *E. coli* DH5α(pNTB1) is shown in Fig. 2. As shown in Fig. 2, it was revealed that, upon introduction of the decaprenyl diphosphate synthase gene for expression thereof, coenzyme Q₁₀, which is originally not produced in *Escherichia coli*, could now be produced in recombinant *Escherichia coli*.

The thus-obtained recombinant *Escherichia coli* DH5α(pNTB1) has been deposited with the National Institute of Advanced Industrial Science and Technology International Patent Organism Depositary, Central 6, 1-1-1 Higashi, Tsukuba, Ibaraki, Japan as of March 16, 200i (the accession number FERM BP-7512).

### INDUSTRIAL APPLICABILITY

A gene encoding decaprenyl diphosphate synthase, which is the key enzyme in the biosynthesis of coenzyme Q₁₀, was isolated from a fungal species belonging to the genus *Bulleromyces* and it was sequenced. This could successfully be introduced in *Escherichia coli* for expression thereof. Furthermore, improvements in gene sequence successfully resulted in production in significant amounts. By using the method of the invention, it becomes possible to efficiently produce coenzyme Q₁₀, which is in use as a drug, among others.

## Claims

1. A DNA of the following (a), (b) or (c):
(a) a DNA whose base sequence is as described under SEQ ID NO:1;
(b) a DNA having a base sequence derived from the base sequence shown under SEQ ID NO:1 by deletion, addition, insertion and/or substitution of one or several bases and
encoding a protein having decaprenyl diphosphate synthase activity;
(c) a DNA capable of hybridizing with a DNA comprising the base sequence shown under SEQ ID NO:1 under a stringent condition and
encoding a protein having decaprenyl diphosphate synthase activity.

2. A protein of the following (d) or (e):
(d) a protein whose amino acid sequence is as described under SEQ ID NO:2;
(e) a protein having an amino acid sequence derived from the amino acid sequence shown under SEQ ID NO:2 by deletion, addition, insertion and/or substitution of one or several amino acid residues and having decaprenyl diphosphate synthase activity.

3. A DNA encoding the protein according to Claim 2.

4. An expression vector resulting from insertion of the DNA according to Claim 1 or 3 into a vector for expression.

5. The expression vector according to Claim 4,
wherein the vector for expression is pUCNT.

6. The expression vector according to Claim 5,
which is pNTB1.

7. A transformant resulting from transformation of a host microorganism with the DNA according to Claim 1 or 3.

8. A transformant resulting from transformation of a host microorganism with the expression vector according to Claim 4, 5 or 6.

9. The transformant according to Claim 7 or 8,
wherein the host microorganism is a strain of *Escherichia coli*.

10. The transformant according to Claim 9,
wherein the *Escherichia coli* strain is *Escherichia coli* DH5α.

11. The transformant according to Claim 10,
which is *E. coli* DH5α(pNTB1) (FRPM BP-7512).

12. A process for producing coenzyme Q₁₀,
which comprises cultivating the transformant according to Claim 7, 8, 9, 10 or 11 in a medium and recovering coenzyme Q₁₀ thus formed and accumulated in the medium.
